# EUROPEAN PATENT SPECIFICATION

(11) **EP 1 488 006 B1**
(45) Date of publication and mention of the grant of the patent: **28.05.2008**
(21) Application number: 03744383.5
(22) Date of filing: 20.03.2003
(51) Int. Cl.: C12Q 1/68, B01J 13/02, B01J 13/04

(54) **MICROCAPSULES WITH CONTROLABLE PERMEABILITY ENCAPSULATING A NUCLEIC ACID AMPLIFICATION REACTION MIXTURE AND THEIR USE AS REACTION COMPARTMENTS FOR PARALLELS REACTIONS**
MIKROKAPSELN MIT KONTROLLIERTER DURCHLÄSSIGKEIT DIE EIN NUKLEINSÄUREAMPLIFIZIERUNGSREAKTIONSGEMISCH ENTHALTEN UND DEREN BENUTZUNG ALS REAKTIONSGEFÄSS FÜR PARALLELE REAKTIONEN
MICROCAPSULES ENCAPSULANT UN MELANGE DE REACTION D'AMPLIFICATION DE L'ACIDE NUCLEIQUE ET UTILISATION DE CELLES-CI EN TANT QUE COMPARTIMENTS DE REACTION POUR DES REACTIONS PARALLELES

(30) Priority: 20.03.2002 EP 02006279
(43) Date of publication of application: 22.12.2004
(73) Proprietor: innovativeBio.Biz, Kowloon, Hong Kong (CN)
(72) Inventor: TRAU, Dieter, Hong Kong SAR (CN); RENNEBERG, Reinhard, Hong Kong University of S.&T., Hong Kong SAR (CN); MAK, Wing Cheung, Hong Kong SAR (CN)
(74) Representative: Dey, Michael
(86) International application number: PCT/EP2003/002926
(87) International publication number: WO 2003/078659

(56) References cited:
- EP-A- 0 572 057
- EP-A- 1 116 516
- WO-A-00/52180
- WO-A-01/34842
- WO-A-02/17888
- WO-A-93/16200
- WO-A-96/00301
- US-B1- 6 251 661
- STRIZHKOV BORIS N ET AL: "PCR amplification on a microarray of gel-immobilized oligonucleotides: Detection of bacterial toxin- and drug-resistant genes and their mutations" BIOTECHNIQUES, EATON PUBLISHING, NATICK, US, vol. 29, no. 4, October 2000 (2000-10), pages 844-857, XP002167651 ISSN: 0736-6205

## Description

The present invention refers to microcapsules of controlled permeability encapsulating a nucleic acid amplification reaction mixture, in particular, a PCR reaction.mixture and providing a compartment to perform nucleic acid polymerization and/or amplification, in particular, a PCR reaction. During the nucleic acid polymerization and/or amplification, in particular, the PCR reaction, high molecular weight nucleic acid product, in particular, a PCR product is accumulated in the interior of capsules that contain polymerase and appropriate templates and primers, while low molecular weight substrate molecules, in particular dNTPs and/or ddNTPs, permeate into the capsule from the outside during the reaction. The invention also describes the encapsulation of a nucleic acid amplification reaction mixture, in particular, a PCR reaction mixture into microcapsules and the use of such capsules to carry out multiple nucleic acid polymerizations and/or amplifications, in particular, PCR reactions simultaneously. The described capsules create a compartment that separates individual nucleic acid polymerization and/or amplification reactions, in particular, PCR reactions. Furthermore the invention describes the detection, isolation and analysis of capsules that contain a nucleic acid product, in particular, a PCR product. Application examples of simultaneous multiple nucleic acid polymerizations and/or amplifications, in particular, PCR for analytical purpose are provided.

In the state of the art PCR reactions are carried out in reaction tubes or micro titer plates or in glass capillaries. The reaction volume varies from 50 to 200 µl for tubes to 10 to 100 µl for microtiter plates and below to 20 µl for glass capillaries used by the latest and most advanced PCR cycler from Roche (LightCycler). The parallelism of PCR is restricted to about 48 reactions by using tubes and 96 to less than 2000 reactions by using high-density microtiter plates. Instruments using glass capillaries can perform 96 reactions in parallel. This numbers are still too small to perform screening projects, e.g. in pharmacological research in a appropriate time. The advantage of the latest PCR cycler models (e.g. Roche LightCycler) is that they are capable to perform Real-time PCR. In Real-time PCR, information about the generation of PCR products and sequence information can be obtained for individual reactions in real-time. In current PCR technique, individual PCR reactions are separated by using tubes, capillaries or wells. These tubes capillaries or wells are made from plastic ware (PS or PP) or glass. To perform a higher number of reactions in parallel, the integration density must be increased and the reaction volume must be decreased. To increase the number of parallel PCR reactions with current technology has limited chance to succeed. The standard microtiter plate format is already at the edge of its integration with 1539 cavities per plate. Furthermore, the filling of this large number of cavities becomes a substantial problem.

By using state of the art PCR equipment about 48 to 1539 PCR reactions are carried out per run in parallel. Special instruments can handle more than one PCR microtiter plate to increase the throughput. However, the increase is only about one order of magnitude and the instruments need big lab space.

T. Oberholzer et al., Chemistry & biology, 2 (1995), 677-682 describe a polymerase chain reaction in liposomes. However, liposomes are unpermeable to the components of a PCR reaction so that all components must initially be present within the liposomes thus greatly limiting the practical application.

US-B1-6 251 661 discloses the use of non-permeable capsules for nucleic acid amplification. EP-A-1 116 516 discloses a permeable capsule for the controlled release of an encapsulated material.

Therefore, it was an object of the present invention to provide an improved nucleic acid amplification process. In particular, it was an object of the present invention to provide a nucleic acid amplification process which allows for the parallel performance of a large number of parallel nucleic acid amplification reactions.

This object is achieved according to the invention by a nucleic acid amplification process comprising the steps
(a) providing a nucleic acid amplification reaction mixture,
(b) encapsulating said reaction mixture into capsules being permeable for low molecular weight molecules and not permeable for high molecular weight molecules and thereby forming capsules comprising constituents of the reaction mixture, and
(c) performing at least one amplification step and thereby amplifying a nucleic acid within one or more of the capsules.

The present invention describes a method which allows carrying out up to 10⁵ to 10¹¹ nucleic acid amplifications, in particular, PCR reactions in parallel.

The present invention is based on the encapsulation of a nucleic acid amplification reaction mixture, in particular, a PCR reaction mixture into microcapsules with high permeability for low molecular weight molecules and no permeability for high molecular weight molecules. The capsules create a compartment to separate individual nucleic acid amplifications, in particular, PCR reactions from each other. During the nucleic acid amplification, high molecular weight nucleic acid amplification product, in particular, a PCR product is accumulated in the interior of capsules that contain polymerase and appropriate templates and primers, low molecular weight substrate molecules, in particular dNTPs, permeate into the capsule from the outside. Low molecular weight molecules according to the invention have a molecular weight of less than 2000 Da, in particular less than 1500 Da and preferably less than 1200 Da and comprise e.g. dNTPs, ddNTPs and labeled, in particular fluorescent labeled nucleotides. High molecular weight molecules have accordingly a molecular weight of greater than 2000 Da, in particular greater than 2500 Da and preferably greater than 3000 Da and comprise e.g. polymerase, primers, templates and amplification products.

Typical capsule diameters are in the range of 50 nm to 1000 µm, preferably 500 nm to 100 µm, and most preferably 40 to 60 µm. Capsules are preferably dispersed in a buffer, e.g. a PCR buffer (containing dNTPs) with a density of about 10⁵ to 10¹¹ capsules per ml. By using microcapsules encapsulating a nucleic acid amplification reaction mixture, in particular, a PCR reaction mixture a very large number of parallel reactions in a small volume is possible. In comparison to prior art technology a microcapsule is used to separate individual nucleic acid amplifications, in particular, PCR reactions and not a tube or well made from plastic ware or glass. By using the present invention a number of up to about 10⁵ to 10¹¹ parallel nucleic acid amplifications, in particular; PCR reactions is possible (depending on capsule size and density) in a single nucleic acid amplification, in particular, a PCR run. A preferred application is the isolation of single DNA strands from a complex DNA mixture, as well as screening, sequencing and construction of DNA libraries. Flow cytometry is a powerful method to analyze and separate capsules that contain nucleic acid amplification product, in particular, a PCR product. The present invention provides a method for mass screening with a throughput that is not realizable with prior art techniques.

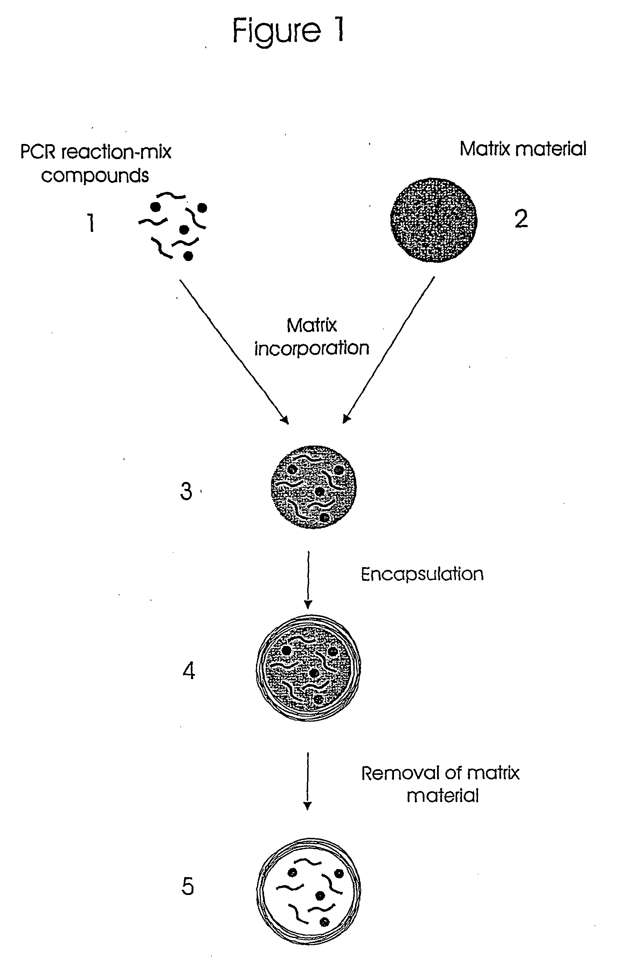

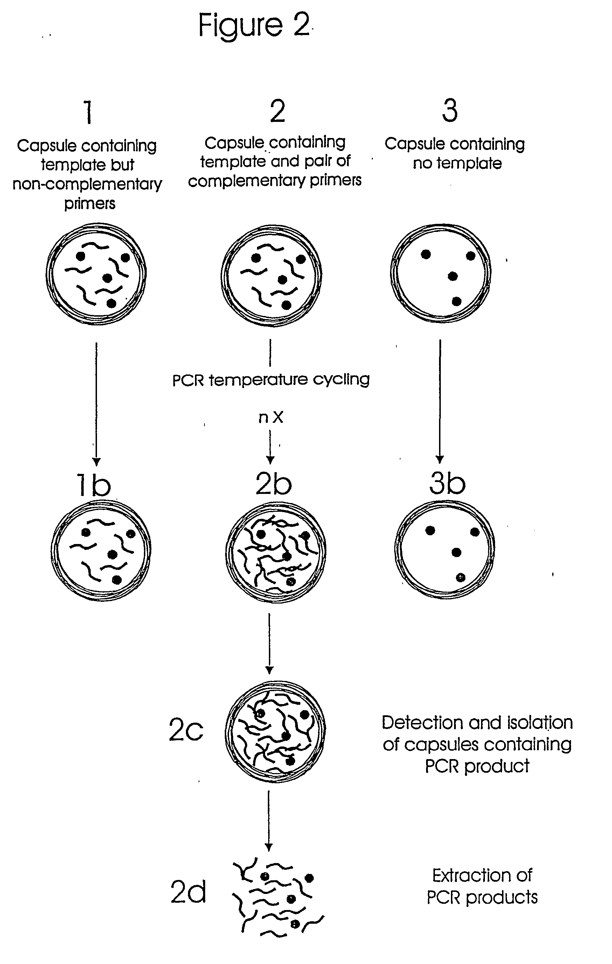

The present invention refers to microcapsules encapsulating a nucleic acid amplification reaction mixture, in particular, a PCR reaction mixture and providing a compartment to perform a nucleic acid amplification, in particular, a PCR reaction. In a first step, a nucleic acid amplification mixture is provided. This mixture may contain all or some of the reaction partners or components required for performing a nucleic acid amplification mixture. Additional components may be added after capsule formation. Preferably, a PCR reaction mixture consisting of: polymerase and/or primers and/or templates and/or buffer substances and/or dNTPs and/or enhancers and/or DNA probes are provided and, more preferably, mixed with a microparticle-forming matrix material (e.g., agarose) and microparticles of the matrix material with incorporated PCR reaction mixture are formed (e.g., by ionotropic gel formation of alginate or agarose) (Figure 1 /2 3). An alternative method is the incorporation of the reaction mixture into a porous microparticle as a matrix. A detailed description of the method is given in the section EXAMPLES.

In a second step, the nucleic acid amplification mixture is encapsulated. In this step capsules comprising reaction partners or constituents of the reaction mixture are formed. While the reaction mixture itself can be encapsulated, it is preferred to encapsulate matrix particles containing the reaction mixture. In this embodiment the constituents of the nucleic acid amplification reaction mixture are incorporated into a matrix material prior to encapsulation. Suitable matrix materials are, for example, porous microparticles, wax-like substances, agarose, alginates, polysaccharides, a polypeptide, fatty acids, salts of fatty acids, polyvinylpyrrolidone (PVP) or the matrix is ice, or mixtures thereof. In a preferred embodiment, matrix material (e.g., agarose, alginate, microparticle) with incorporated PCR reaction mixture is subsequently encapsulated (Figure 1 /2 4). A variety of methods can be used for encapsulation, e.g. Layer-by-Layer technology or interfacial polymerization or cavaciation. The permeability of the capsule can be controlled in a way that small molecules (e.g., dNTPs, ddNTPs, short PCR primers) can permeate through the capsule wall but larger molecules (e.g., DNA templates, large PCR primers, PCR products, polymerase) cannot pass the capsule.

A requirement for using the Layer-by-Layer technology for encapsulation is a charged template (the material to be encapsulated). Agarose and alginate microparticles are negatively charged and a suitable template. Porous microparticles are available with negative or positive surface charges. By using the Layer-by-Layer encapsulation technique a permeability control is achieved by controlling the number of polyelectrolyte layers, the polyelectrolyte material, the molecular weight of the polyelectrolyte and the crosslinking.

Optionally, the matrix material is dissolved or removed out of the capsule (Figure 1/2 5) after the encapsulation process is performed. E.g., for alginate-based matrix material by an exchange to a calcium free buffer. Dissolving or removing of the matrix material can be performed, e.g. by dissolution with solvents, by chemical means such as oxidation, or by high temperatures during PCR cycles. Agarose matrix material will melt under PCR temperature cycling conditions resulting in high permeability of molecules within the capsule.

Next, at least one amplification step, e.g. PCR cycling, is performed to amplify a nucleic acid within one or more of the capsules. Prior to the amplification step preferably non-encapsulated reaction mixture compounds are separated from the capsules, e.g. by centrifugation, filtration, dialysis, or destruction with nucleases and/or proteases. The dispersion of microcapsules in buffer, e.g. a PCR buffer is used for nucleic acid amplification, in particular, PCR experiments. PCR cycling is achieved by placing a reaction tube (or micro titer plate) containing microcapsules dispersion in a conventional PCR cycler programmed with standard PCR cycling temperature profiles.

A particular advantage of the process according to the invention is that components, constituents or reactants of the nucleic acid amplification reaction can be introduced selective into the capsules during and/or before the amplification and/or polymerization reaction is performed in a continuous or batchwise manner. Preferably, the permeability of the capsules is controlled to allow selective diffusion of starting materials into the capsules such as dNTPs but restrict the out-diffusion of reaction products or templates. This allows for a continuous or batchwise supplementation of one or several starting materials into the capsules and for further enrichment of the desired product. Thus, the performance is not limited to initially present reactants.

For example, continuous selective exchange can take place during amplification. Layer-by-Layer encapsulated nucleic acid amplification reaction mixtures, in particular, PCR mixtures can exchange low molecular reactants, in particular dNTPs, with the extra capsular media. The dNTPs can easily diffuse through the capsule wall. Thus, dNTPs can be added to the reaction mixture. The optimal dNTP concentration can be kept constant in the interior of the capsule over a long period of time. The nucleic acid amplification or polymerization, in particular, a PCR reaction is therefore not limited to the building block (e.g. dNTPs) material in the capsule.

If preferably fluorescent labeled dNTPs are used, amplification products are labeled and capsules containing amplification products can be easily identified. If 4 color fluorescent labeled ddNTPs are used, polymerization products are fluorescent labeled regarding to their 3'-end base.

For example, batchwise selective exchange can take place before or during amplification. Constituents, components or reactants can be transferred into the capsule by a triggered change in capsule wall permeability. The capsule wall permeability, for example, can be varied or changed by a pH change, e.g. pH dependent swelling or shrinking of hydrogel capsule materials, or an ionic strength change of the reaction medium. In a more preferred process, capsules are constructed of 3 to 6 layers of thiolated polyelectrolytes in a Layer-by-Layer approach with an outer non-thiolated layer. In the presence of oxygen, thiol groups form disulfide bridges leading to a crosslinking of polyelectrolyte layers resulting in lower permeability. Addition of reductive agent (e.g. dithiotritol, DTT) results in a reduction of disulfide groups to thiols and reverses the process, resulting in higher capsule permeability. In addition, this principle can be applied to create stable crosslinked capsule to perform PCR cycling and to intentionally break such capsules e.g., to release DNA products to perform electrophoresis experiments.

Continuous selective exchange during amplification and batchwise addition of reactants can be combined e.g. first a DNA sequence is amplified by PCR using continuous inflow of dNTPs followed by batchwise addition of fluorescent labeled ddNTPs to generate 3'-end fluorescent labeled products.

Preferably, the process according to the invention comprises the further step
(d) detecting capsules containing a nucleic acid product. This step can be performed e.g. by using flow cytometry or microscopy.

In a further preferred embodiment, one or more components of the nucleic acid amplification reaction mixture and/or of the capsule material carry a label. Suitable labels are e.g. fluorophores, quantum dots, radioisotopes, dyes or NMR active isotopes or nanoparticles. In particular, the labels are selected to enable the encoding of individual capsules and/or the encoding of distinct reaction products.

Positive capsules, i.e. capsules containing nucleic acid product, are preferably separated from the reaction mixture. For example, capsules containing nucleic acid products can be detected by the physical signature caused by a label or by dyes staining DNA or by UV adsorption measurement of DNA and are separated and isolated from the capsule suspension. The nucleic acid product of said capsules can then be analyzed or extracted for further use. Preferably a PCR product is extracted from separated capsules or a single capsule.

Oberholzer et al. investigated in the origin of live and performed a PCR reaction in liposomes (Chemistry & Biology, 2:677-682, 1995). Liposome encapsulated nucleic acid amplification reaction mixtures however, cannot exchange DNA, primer, staining dyes or dNTP material with the external media. The lipid bilayers of liposomes do not allow diffusion of charged molecules (e.g. all PCR mixture compounds are charged and/or macromolecular). Therefore the PCR reaction comes to still stand after most of the dNTPs are used up. Also capsules with PCR product cannot be distinguished from capsules without PCR product.

In the current invention preferably labeled starting materials, e.g. labeled dNTPs or labeled ddNTPS are used resulting in labeled nucleic acids, e.g. in a fluorescent labeled PCR product, which is accumulated in capsules with PCR reaction. Such capsules can be easily detected e.g. due to their fluorescent intensity, and isolated, e.g. by UV or fluorescent detection.

In a preferred embodiment, the PCR reaction takes only place in capsules that contain a template and a complementary pair of primers (Figure 2/2 2 and 2b). Other capsules (Figure 2/2 1 and 3) will not build PCR product. By using fluorescent-labeled dNTPs in the PCR experiment, PCR product is labeled with fluorescent molecules. Fluorescence intensity increases for capsules with PCR reaction ("positive capsules") during the PCR cycling. An alternative way is the use of intercalating reporter dyes. These dyes (e.g., ethidium bromide, SYBR Green I) increase in fluorescent light intensity while intercalating with double stranded DNA (= PCR product). Sequence information of nucleic acid amplification products, in particular, PCR products can be obtained by using hybridization probes (e.g., Tag Man system or molecular beacons).

Positive capsules can be detected by flow cytometry and separated from the bulk by flow sorting. Modern flow cytometry instruments are capable of analyzing 70 000 particles per second with high accuracy. A microcapsule suspension with 10⁹ capsules needs an analyzing time of about 4 hours. Modern instruments are equipped with multicolor analysis. This method can be used to identify encoded capsules or to track single capsules in complex mixtures. By using flow cytometry the present invention provides a method for mass screening with extremely high throughput.

In another embodiment of the invention, microcapsules encapsulating a nucleic acid amplification reaction mixture, in particular, a PCR reaction mixture are fixed onto a 2-dimensional surface. The location of individual capsules is determined by a video camera. A laser that illuminates the fixed capsules introduces energy to heat up individual capsules to a desired PCR cycling temperature. The laser scans over the surface (e.g., by using electro movable mirrors and optic) and addresses single capsules or a certain surface area. Different PCR temperature profiles are applicable for single capsules or a certain surface area. The capsule temperature is controllable by the laser power and the illumination time. Laser power adsorption can be increased by dyes incorporated into the capsule wall. In addition dyes can be used to locate capsule positions. By using fluorescent labeled dNTPs the increase of PCR product in capsules is online measurable by an increase in fluorescent light intensity originated from capsules containing PCR product. This allows real time PCR of individual capsules. Optimal PCR temperature profiles for individual capsules can be evolved by the sequential application of different PCR temperature profiles and real-time measurement of fluorescent intensity increase. By using an intelligent feedback loop temperature profiles can be optimized and evolved for maximum amplification or specificity.

Most preferred in the process according to the invention, the nucleic acid amplification reaction is a polymerase chain reaction (PCR). In this case the process preferably comprises the steps:
(i) providing a PCR reaction mixture consisting of polymerase and/or dNTPs and/or buffer substances and/or enhancers and/or primers and/or templates and/or DNA probes and/or matrix material,
(ii) encapsulating said PCR reaction mixture in capsules being permeable for low molecular weight molecules and not permeable for high molecular weight molecules and thereby forming capsules comprising constituents of the reaction mixture and
(iii) performing at least one amplification step comprising PCR temperature cycling and thereby amplifying a nucleic acid within one or more of the capsules.

The present invention further relates to a capsule encapsulating a nucleic acid polymerizations and/or amplification reaction mixture, in particular, a PCR reaction mixture and/or a nucleic acid amplification product, in particular, a PCR product. These capsules can be advantageously used for a variety of applications. Besides or after the use as reaction compartment for parallel nucleic acid amplification and/or polymerization reactions they can be applied for the analysis and/or screening of nucleic acids or artificial derivatives of nucleic acids, for the production of nucleic acid libraries, for nucleic acid sequencing, in bioassays, or in real-time PCR.

In another embodiment of the invention, microcapsules encapsulating a nucleic acid amplification reaction mixture, in particular, a PCR reaction mixture are used to construct a genomic DNA library comprising the steps:
(i) isolation of genomic DNA,
(ii) partial digestion of the genomic DNA with restriction enzymes into fragments of 500 to 50 kb having sticky ends (e.g. with Sau3A),
(iii) extending both ends of the genomic DNA fragments with a known DNA by hybridization and ligation to create recombinant DNA, (e.g. by using λ phage DNA treated with BamHl nuclease to create sticky ends complementary to the genomic DNA fragments. Removal of the out cut middle part (= replaceable region) of λ phage DNA. Hybridization of λ phage DNA fragments with genomic DNA fragments. Ligation of fragments creates recombinant DNA).
(iv) preparation of a PCR reaction mixture with recombinant DNA as a template and a primer pair complementary to a region that amplify the genomic DNA fragment,
(v) encapsulation of said mixture into microcapsules, resulting in capsules carrying statistically one copy per capsule and
(vi) performing nucleic acid amplification.

In another embodiment of the invention, microcapsules encapsulating a nucleic acid amplification reaction mixture, in particular, a PCR reaction mixture are used to construct a cDNA library comprising the steps:
(i) isolation of mRNA,
(ii) hybridization of poly(T) oligonucleotide onto the mRNA,
(iii) elongation of the poly (T) primer with reverse transcriptase,
(iv) hydrolyzation of the mRNA with alkali resulting in single,stranded cDNA,
(v) Addition of a oligonucleotide tail, e.g. poly(G), onto the 3' terminus with terminal transferase,
(vi) preparation of a PCR reaction mixture with cDNA as a template and a primer pair that allows PCR amplification of the coding region, e.g., poly(T) and poly(C),
(vii) encapsulation of said mixture into microcapsules, resulting in capsules carrying statistically one copy cDNA per capsule, and
(viii) performing nucleic acid amplification.

In another embodiment of the invention, the nucleic acid product, in particular a PCR product, is analyzed parallel in multiple capsules by using gel chromatography comprising the steps:
(i) embedding capsules filled with nucleic acid product into a thin gel of agarose or polyacrylamide,
(ii) rendering the capsule wall permeable for the nucleic acid product, by chemical or physical means, resulting in release of the nucleic acids from capsules,
(iii) applying an electrical potential to carry out gel electrophoresis,
(iv) detection of band patterns created from individual capsules, and
(v) computing the lengths of DNA fragments from calibrations obtained with capsules filled with DNA ladder.

In another embodiment of the invention, microcapsules encapsulating a nucleic acid reaction mixture, in particular, a PCR reaction mixture are used to sequence DNA or genomic DNA or cDNA comprising the steps:
(i) performing the steps (i) to (v) described for the construction of DNA libraries or steps (i) to (vii) described for the construction of cDNA libraries, resulting in a preferable length of 500 bp of DNA fragments, but using a primer pair with one primer in excess of another, e.g. at a ratio of 2:1 to 1000:1.
(ii) performing PCR until the primer with the lower concentration is used up.
(iii) adding 4 color fluorescent labeled 2',3'-dideoxynucleoside triphosphates (ddNTPs) in a concentration of about 1 /500, e.g. 1 /100 to 1 /1000, of the dNTP concentration to the suspension of microcapsules and performing elongation of the primer with excess concentration,
(iv) performing the steps (i) to (iii) described for the analysis of nucleic acid capsule content by gel electrophoresis,
(v) detection of the band patterns created from individual capsules, at the wavelengths of the four corresponding dyes, and
(vi) computing the sequence of the DNA fragments and the entire genome.

In addition to the described procedures for the construction of DNA libraries, electrophoresis and sequencing the following modification can be made. For the construction of DNA or genomic libraries: The digestion can be carried out with any nuclease that created sticky ends for extension of the dsDNA with a double stranded DNA tail: E.g., by using BamHl the nuclease cut at 5'-G^GATC-NNN-3' // 3'-NNN-CTAG^G-5' and sticky ends of the sequence 5'-GATC-NNN-3' will be formed. Extension can be carried out by hybridization and ligation with dsDNA having a sticky end 5'-GATC-Nn-3' (N = CGAT, n = 1 to 25). This procedure will create identical sequences at the 3'-ends of the DNA molecules. One primer can be used to perform PCR to amplify the two strands.

For the performance of the electrophoretic analysis: The release of nucleic acid product from microcapsules can be achieved e.g., by using disulfide cross linked capsules, described previously. In the gel electrophoresis experiment, each capsule needs at least 50 µm x 5 cm gel area. About 24000 individual electrophoresis experiments can be carried out on an A4 size gel. Representing sequence information of about 10⁷ bases. For plasmids, viruses and bacterium chromosome about 1 A4 gel is ne,eded, for the human genome with 3 x 10⁹ bp about 250 A4 gels are needed and for one human chromosome about 6 gels are needed. The process according to the invention has the potential to sequence a genome in about 1 day.

For the performance of the sequencing: To sequence a DNA library the DNA fragments are required to have two different sequences at their 3'-end. This can be achieved by using a hybridization/ligation to a plasmid. This allows the hybridization of different primers onto the 3'-ends. To generate fragments with fluorescent labeled 3'-end from only one strand either at the coding or non coding strand, an excess of one primer is used. Therefore, the two primers are added at a ratio of 1:2 to 1:1000. A PCR can be carried out until the primer with the lower concentration is used up. Then, the primer in excess is elongated at the presence of ddNTPs. The method can be performed with the forward or the backward primer in excess. By carrying out two experiments, one using the forward and the other using the backward primer in excess, two data sets of complementary sequence can be generated. The elongation of the primer in excess can be repeated several times by using a temperature cycling protocol similar to PCR.

Further, the invention comprises a kit for the production of the inventive capsules or the performance of the inventive processes containing at least one starting material necessary for performing the processes.

The invention is further illustrated in the following figures and examples.

### FIGURES

Figure 1 shows the preparation of capsules encapsulating a PCR reaction mixture. The reaction mixture compounds (Fig 1/2 1) are consisting of dNTPs and/or buffer substances and/or enhancers and/or primers and/or templates and/or polymerase. Prior to their encapsulation the mixture compounds are incorporated into a matrix material (Fig. 1/2 2) resulting in micro particles of matrix material with incorporated reaction mixture (Fig1/2 3). Encapsulation is carried out by using e.g. a layer-by-layer process (Fig. 1/2 4). Subsequently the matrix material is removed leaving the reaction mixture compounds encapsulated into a micro scale capsule (Fig 1/2 5).

Figure 2 shows the application of the encapsulated PCR reaction mixture. The template and/or primers are present in the capsule (Figure 2/2 1-3). A PCR product is generated only in capsules that contain a template complementary to a reverse and forward PCR primer (Figure 2/2 2b). Capsules that contain no template or a template but non-complementary primers cannot generate a PCR product (Figure 2/2 1b and 3b). Capsules that contain a PCR product are identified e.g. by fluorescent or UV spectroscopy and separated (Figure 2/2 2c). PCR products and templates are extracted from capsules or from a single capsule for further studies (Figure 2/2 2d).

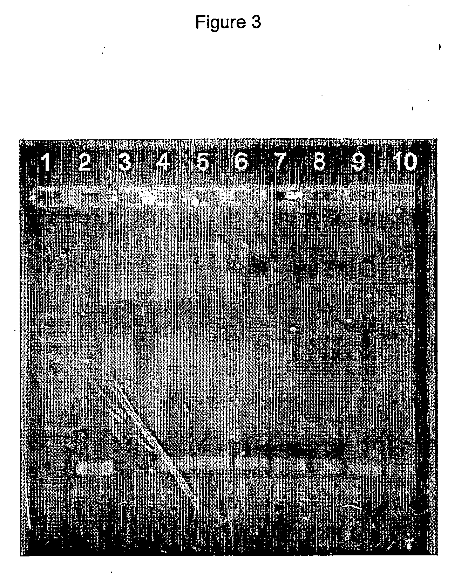

Figure 3 shows that PCR reactions can be successfully performed in agarose gel matrix. Lane: 1 = Ladder (100 bp), 2 = Positive control, 3 = Negative control, 4 = Pos. contr. + 0.1 % agarose, 5 = Pos. contr. + 0.2 % agarose, 6 = Pos. contr. + 0.5 % agarose, 7 = Pos. contr. + 0.6 % agarose, 8 = Pos. contr. + 0.7 % agarose, 9 = Pos. contr. + 0.8 % agarose, 10 = Pos. contr. + 1.0 % agarose, all at a Mg concentration of 1.5 mM.

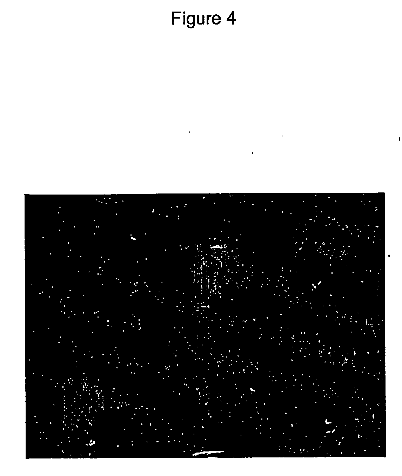

Figure 4 shows microbeads prepared from agarose (about 50 µm diameter). 4a) Phase contrast micrograph shows the morphology of agarose microbeads. 4b) Fluorescent micrograph (FITC filter) shows the microcapsule of beads prepared with FITC labeled PAH. 4c) Fluorescent micrograph (Ethidium filter) shows ethidium bromide stained dsDNA in the interior of microbeads.

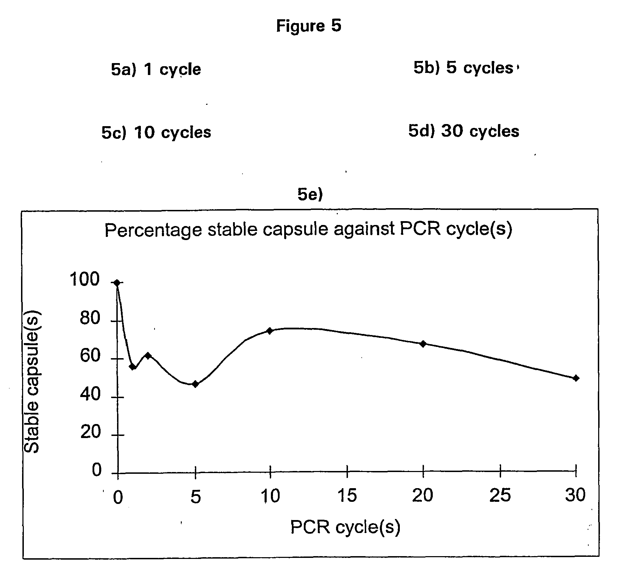

Figure 5 shows the stability of (PAH/PSS)6 encapsulated agarose microbeads (about 50 µm diameter) under PCR temperature cycling conditions (0.5 min at 95 degree, 1 minute at 60 degree and 2 minute at 72 degree). 5a to 5d) micrographs of encapsulated beads after 1, 5, 20 and 30 cycles. 5e) percentage of stable capsules versus PCR cycle number.

### EXAMPLES

Materials: Polycation, poly(allylamine hydrochloride) (PAH), Mw15,000, and polyanion, poly(sodium 4-styrenesulfonate) (PSS), Mw 70,000 from Aldrich. Sodium Alginate from (Manugel) Alginate Industry GmbH. PCR reagents and polymerase from Promega. Templates and primers from Synthetic Genetics.

### Example I: Preparation of capsules encapsulating a PCR reaction mixture

A DNA template dilution (in 1 ml 1 x PCR buffer) with a density of 10³ to 10¹² template molecules per ml was prepared. Taq DNA polymerase (25 U/ml), 0.8 µM of each primer (optional, fluorescently labeled) and 200 µg/m) BSA was added to the template solution.
A) By using a porous microparticle: The solution from above was a mixed with a suspension of 10³ to 10¹² porous micro particles (0.5 to 10 µm in diameter, pore size 5-200 nm) in 100 µl water. The mixture was incubated over night to archive a diffusion of PCR mixture compounds into particle pores (some material may also adsorb onto the particle surface). The particle suspension was centrifuged and the supernatant was discarded. The particles were immediately coated with polyelectrolyte (5 mg PAH in 1 ml 1x PCR buffer). Then the supernatant was removed by centrifugation followed by three cycles of washing with 1 xPCR buffer to remove the excess non-adsorbed polyelectrolyte. Then 1 ml of oppositely charged polyelectrolyte solution (5 mg PSS in 1 ml 1 x PCR buffer) was added to form a consecutive layer on the particle surface. The centrifugation/washing steps and the consequent adsorption of oppositely charged polyelectrolytes were repeated until the desired number of layers is assembled (4-8).
B) By using a matrix material: I) Alginate: 60 mg sodium alginate was diluted in the DNA template solution from above. The solution was transferred into a disperser. An aerosol was created by the disperser and sprayed into a 2 % CaCl₂ solution. After 10 minutes incubation the particle suspension was centrifuges and the supernatant was discarded. The negatively charged alginate particles were coated with polyelectrolyte described under Examples I A but with the addition of 0.5 % CaCI₂ in the coating buffer. The solid alginate core was dissolved by using a calcium free buffer with high sodium concentration in the last step. Optional capsules were treated with homo-bis-functional crosslinking reagent to increase capsule stability.

II) Agarose: 1 % low melting agarose (40 degree melting point) was added to the DNA template solution from above and warmed to 45 degree. 100 µl of this solution was added to 2 mL pre-warmed (45 degree) oil. The mixture was shacked to form an emulsion with a droplet diameter of about 50 µm. The emulsion was poured into 10 ml of cold (4 degree) PAH solution (5 mg/mL) and was stirred for some minutes (1 to 20 min). The agarose microbeads were harvested by centrifugation (1200 g for 10 min). The oil phase was removed and the microbeads were washed with cold buffer. The beads were consecutively coated with multiple layers of PSS and PAH.

### Example II: Performing of PCR temperature cycling and identification of capsules containing PCR product

Capsule suspensions with 10³ to 10¹² capsules per ml are diluted with 9 parts PCR buffer containing 2 mM magnesium chloride, 0.2 mM of each dNTP (fluorescent labeled), 1× Taq buffer. PCR cycling is performed in microreaction tubes (50-200 µl per tube) or in microtiter plates (10-100 µl per well) by using conventional cycler.
The PCR cycle set-point temperatures were set as follows: Initial denaturation 90(C for 300 s (Denaturation 92(C for 60 s; Annealing 50(C for 60 s; Elongation 65(C for 60 s) x 15 to 35 cycles.
A flow cytometer with fluorescent detection and a cell sorter are used to identify and to separate capsules containing PCR product. The resulting suspension of capsules containing PCR product was further analysis. For example: the suspension volume was 1 ml and contains 1000 capsules. The suspension was diluted to 750 capsules per milliliter and 10 µl aliquots were dispensed into each well of a 1536 well microtiter plate (Greiner) resulting in a density of about 0.5 capsules per well (Statistically a very high chance of not more than 1 capsule per well is achieved). After rupturing of capsules (addition of ~100 µl 1 M NaOH, 1 min incubation, addition of the same volume 1 M HCL) PCR products originated from a single strand of template are present in particular wells. Wells containing DNA (=fluorescent labeled PCR product) are easy to identify by fluorescent spectroscopy.

### Example III: Applications in nuclear acid analysis and screening

### A) Isolating a single target DNA molecule from a complex mixture of DNA molecules:

A complex mixture of DNA molecules (~10¹⁰ molecules) was diluted and encapsulated together with the PCR reaction mixture described above, leading to a suspension of about 10²⁰ capsules. Statistically 0.5 to 1 molecule should be present in one capsule. A primer pair designed to amplify a region of the target sequence was added to the template solution prior encapsulation (0.8 µM). The PCR reaction and the isolation of capsules containing PCR product was performed as described in Example II. A positive capsule containing PCR product contains the target DNA molecule (minimum 1 copy) as well. A single positive capsule containing at least one copy of the target DNA can be used for further analysis. The capsule can be destructed and additional target sequences can be identified on the isolated target DNA by PCR.

## Claims

1. A nucleic acid amplification and detection process comprising the steps
(a) providing a nucleic acid amplification reaction mixture,
(b) encapsulating said reaction mixture by 10³ to 10¹² capsules being permeable to low molecular weight molecules having a molecular weight of less than 2,000 Da and not permeable to high molecular weight molecules having a molecular weight of greater than 2,000 Da and thereby forming capsules comprising constituents of the reaction mixture, and
(c) dispersing the capsules in a buffer comprising labeled building blocks,
(d) performing at least one amplification or/and polymerization step and thereby amplifying nucleic acids within one or more of the capsules containing a complementary template and primer pair, whereby building blocks of the amplification or/and polymerization reaction permeate into and are introduced into the capsules during the reaction, and accumulating a nucleic acid product in the capsules, and
(e) detecting capsules containing a nucleic acid product by using the signature caused by the label of the labeled building blocks.

2. The process according to claim 1, wherein the building blocks which are introduced into the capsules during the reaction are selected from dNTPs and ddNTPs.

3. The process according to any of the preceding claims, wherein labeled dNTPs or labeled ddNTPs are introduced into the capsules during the reaction.

4. The process according to any of the preceding claims, wherein 10⁵ to 10¹¹ nucleic acid amplifications are carried out in parallel.

5. The process according to any of the preceding claims, wherein the high molecular weight molecules which cannot pass the capsule are selected from DNA templates, PCR products and polymerase.

6. The process according to any of the preceding claims, further comprising the step
(f) separating capsules containing nucleic acid product from capsules not containing nucleic acid products.

7. The process according to any of the preceding claims, further comprising the step
(g) analyzing or extracting the nucleic acid product.

8. The process according to claim 1, wherein the constituents of the nucleic acid amplification reaction mixture are incorporated into a matrix material.

9. The process according to claim 8, wherein the matrix material is selected from a porous microparticle, a wax-like substance, an alginate, an agarose, a polysaccharide, a polypeptide, a fatty acid, a salt of a fatty acid, polyvinyl pyrrolidone (PVP), or the matrix is ice, or a mixture thereof.

10. The process according to any of the preceding claims, wherein the nucleic acid amplification reaction mixture incorporated into a matrix material is encapsulated using a layer-by-layer process, a polymerization process or/and a cavaciation process.

11. The process according to any of claims 8 to 10, wherein the matrix material is dissolved and/or removed from the capsule with solvents, by chemical means or by physical means such as heat.

12. The process according to claims 8 to 10, wherein the matrix material is melted during temperature cycling.

13. The process of any of the preceding claims, wherein capsules are formed having a diameter from 50 nm to 1000 µm, with a preferred diameter of 40 to 60 µm.

14. The process according to any of the preceding claims, wherein constituents and/or reactants of the nucleic acid amplification reaction mixture are transferred into the capsule by a triggered change in capsule wall permeability.

15. The process according to claim 14, wherein the change in capsule wall permeability is triggered by a pH change or an ionic strength change or a change in capsule cross linking.

16. A process according to claim 14 or 15, wherein the permeability of the capsule is controlled to allow selective diffusion of nucleic acid amplification reaction starting materials into the capsules but restrict the out-diffusion of nucleic acid amplification reaction products and/or templates and/or primers.

17. The process according to any of the preceding claims, wherein non-encapsulated nucleic acid amplification reaction mixture compounds are separated from the capsules or destructed by enzymes prior to the amplification step.

18. The process according to any of the preceding claims, wherein one or more components of the nucleic acid amplification reaction mixture and/or of the capsule material carries a label.

19. The process according to claim 18, wherein the label is selected from a fluorophore, a quantum dot, a radioisotope, a dye, a nanoparticle or an NMR active isotope.

20. The process according to claim 18 or 19, wherein the label enables the encoding of individual capsules.

21. The process according to any of the preceding claims, wherein capsules containing nucleic acid products are detected by the physical signature caused by a label or by UV adsorption measurement of DNA, or by dyes staining the DNA, and are separated and isolated from the capsule suspension.

22. The process according to any of the preceding claims for the analysis and/or screening of nucleic acids or artificial derivatives of nucleic acids.

23. The process according to any of the preceding claims for the production of a nucleic acid library.

24. The process of any of the preceding claims employed in a bioassay.

25. The process according to any of the preceding claims, **characterized in that** the nucleic acid amplification reaction is a polymerase chain reaction (PCR).

26. The process according to claim 25, comprising the steps
(i) providing a PCR reaction mixture consisting of polymerase and/or dNTPs and/or buffer substances and/or enhancers and/or primers and/or templates and/or DNA probes and/or matrix material,
(ii) encapsulating said PCR reaction mixture and thereby forming capsules comprising constituents of the reaction mixture and
(iii) performing at least one amplification step comprising PCR temperature cycling and thereby amplifying a nucleic acid within one or more of the capsules.

27. The process according to any of claims 14-16 or 25-26, wherein the template and/or one or more primers are transferred into the capsule by a triggered change in capsule wall permeability.

28. The process according to any of claims 14-16 or 25-26, wherein the permeability of the capsule is controlled to allow selective diffusion of dNTPs and/or ddNTPs and/or small primers into the capsules but restrict out-diffusion of polymerase, PCR products, templates and large primers during the temperature cycling.

29. The process according to any of claims 25-28, wherein the capsules are dispersed in a PCR buffer.

30. The process according to claim 29, wherein the PCR buffer contains intercalating dyes such as ethidium bromide and/or SYBR Green 1.

31. The process according to any of claims 25-30, wherein the temperature cycling of the capsule suspension is carried out in reaction tubes, multititer plates or glass capillaries, using a thermocycler.

32. The process according to any of the preceding claims, wherein dNTPs and or ddNTPs and/or primers and/or templates and/or DNA probes and/or capsule material carry a label.

33. The process according to any of claims 18-19 or 32, wherein the label enables the encoding of primers and/or templates and/or mixtures thereof.

34. The process according to any of claims 25-33, wherein further enzymes such as reverse transcriptases and/or nucleases and/or ligases are added into the encapsulated PCR reaction mixture.

35. A method of sequencing the nucleic acid content of capsules obtained by any of claims 1-34 comprising the steps:
(i) performing the PCR amplification with an excess of one primer until the primer with the lower concentration is used up.
(ii) adding 4 color fluorescent labeled ddNTPs at an amount of 1:100 to 1:1000 of dNTPs and elongating the excess primer and
(iii) analyzing the nucleic acid capsule content of individual capsules by gel electrophoresis.

## Patentansprüche

1. Verfahren zur Amplifizierung und zum Nachweis von Nukleinsäure, umfassend die Schritte
(a) Bereitstellen einer Reaktionsmischung zur Amplifizierung von Nukleinsäure,
(b) Einkapseln der Reaktionsmischung in 10³ bis 10¹² Kapseln, die für niedermolekulare Moleküle, die ein Molekulargewicht von weniger als 2.000 Da haben, permeabel sind und die für hochmolekulare Moleküle, die ein Molekulargewicht von größer als 2.000 Da haben, nicht permeabel sind, so dass dadurch Kapseln gebildet werden, die Bestandteile des Reaktionsgemischs umfassen, und
(c) Dispergieren der Kapseln in einem Puffer, der markierte Bausteine umfasst,
(d) Ausführen mindestens eines Amplifizierungs- oder/und Polymerisationsschrittes, so dass dadurch Nukleinsäuren innerhalb einer oder mehrerer Kapseln, die ein komplementäres Templat und ein Primerpaar enthalten, amplifiziert werden, wobei Bausteine der Amplifizierungs- oder/und Polymerisationsreaktion während der Reaktion in die Kapseln eindringen und in die Kapseln eingeführt werden, und ein Nukleinsäureprodukt in den Kapseln angereichert wird, und
(e) Nachweisen von Kapseln, die ein Nukleinsäureprodukt enthalten mittels der Signatur, die durch die Markierung der markierten Bausteine erzeugt wird.

2. Verfahren nach Anspruch 1, wobei die Bausteine, die in die Kapseln während der Reaktion eingeführt werden, ausgewählt sind aus dNTPs und ddNTPs.

3. Verfahren nach einem der vorhergehenden Ansprüche, wobei markierte dNTPs oder markierte ddNTPs während der Reaktion in die Kapseln eingeführt werden.

4. Verfahren nach einem der vorhergehenden Ansprüche, wobei 10⁵ bis 10¹¹ Nukleinsäureamplifizierurigen parallel ausgeführt werden.

5. Verfahren nach einem der vorhergehenden Ansprüche, wobei die hochmolekularen Moleküle, die die Kapsel nicht passieren können, ausgewählt sind aus DNA-Templaten, PCR-Produkten und Polymerase.

6. Verfahren nach einem der vorhergehenden Ansprüche, ferner umfassend den Schritt
(f) Trennen der Kapseln, die Nukleinsäureprodukt enthalten, von Kapseln, die keine Nukleinsäureprodukte enthalten.

7. Verfahren nach einem der vorhergehenden Ansprüche, ferner umfassend den Schritt
(g) Untersuchen oder Extrahieren des Nukleinsäureprodukts.

8. Verfahren nach Anspruch 1, wobei die Bestandteile der Reaktionsmischung zur Amplifizierung von Nukleinsäure in ein Matrixmaterial eingebracht werden.

9. Verfahren nach Anspruch 8, wobei das Matrixmaterial ausgewählt ist aus einem porösen Mikropartikel, einer wachsähnlichen Substanz, einem Alginat, einer Agarose, einem Polysaccharid, einem Polypeptid, einer Fettsäure, einem Salz einer Fettsäure, Polyvinylpyrrolidon (PVP) oder die Matrix Eis ist oder eine Mischung davon.

10. Verfahren nach einem der vorhergehenden Ansprüche, wobei die Reaktionsmischung zur Amplifizierung von Nukleinsäure, die in ein Matrixmaterial eingebracht ist, mittels eines Layer-by-layer-Verfahrens, eines Polymerisationsverfahrens oder/und eines Kavatiationsverfahrens eingekapselt wird.

11. Verfahren nach einem der Ansprüche 8 bis 10, wobei das Matrixmaterial von der Kapsel mit Lösungsmitteln, auf chemische Weise oder auf physikalische Weise, wie zum Beispiel Hitze, gelöst und/oder entfernt wird.

12. Verfahren nach den Ansprüche 8 bis 10, wobei das Matrixmaterial während Temperaturzyklen aufgeschmolzen wird.

13. Verfahren nach einem der vorhergehenden Ansprüche, wobei die Kapseln so geformt sind, dass sie einen Durchmesser von 50 nm bis 1000 µm, mit einem bevorzugten Durchmesser von 40 bis 60 µm, aufweisen.

14. Verfahren nach einem der vorhergehenden Ansprüche, wobei die Bestandteile und/oder Reaktanden der Reaktionsmischung zur Amplifizierung von Nukleinsäure durch eine ausgelöste Veränderung der Permeabilität der Kapselwand in die Kapsel transferiert werden

15. Verfahren nach Anspruch 14, wobei die Veränderung in der Permeabilität der Kapselwand durch eine pH-Veränderung oder eine Veränderung der Ionenstärke oder eine Veränderung in der Kapselvernetzung ausgelöst wird.

16. Verfahren nach Anspruch 14 oder 15, wobei die Permeabilität der Kapsel so reguliert wird, dass selektive Diffusion von Reaktionsausgangsmaterialien zur Amplifizierung von Nukleinsäure in die Kapseln möglich ist, allerdings das Herausdiffundieren von Reaktionsprodukten bei der Amplifizierung von Nukleinsäure und/oder von Templaten und/oder von Primern beschränkt ist.

17. Verfahren nach einem der vorhergehenden Ansprüche, wobei nicht eingekapselte Reaktionsmischungsverbindungen zur Amplifizierung von Nukleinsäure von den Kapseln getrennt werden oder durch Enzyme vor dem Amplifizierungsschritt zerstört werden.

18. Verfahren nach einem der vorhergehenden Ansprüche, wobei eine oder mehrere Komponenten der Reaktionsmischung zur Amplifizierung von Nukleinsäure und/oder des Kapselmaterials eine Markierung trägt.

19. Verfahren nach Anspruch 18, wobei die Markierung ausgewählt ist aus einem Fluorophor, einem Quanten Dot, einem Radioisotop, einem Farbstoff, einem Nanopartikel oder einem NMR-aktiven Isotop.

20. Verfahren nach Anspruch 18 oder 19, wobei die Markierung das Kodieren von einzelnen Kapseln ermöglicht.

21. Verfahren nach einem der vorhergehenden Ansprüche, wobei die Kapseln, die Nukleinsäureprodukte enthalten, durch die physikalische Signatur, die durch eine Markierung erzeugt wird, oder durch UV-Adsorptionsmessung von DNA oder durch Einfärben der DNA mit Farbstoffen nachgewiesen werden, und von der Kapselsuspension getrennt und isoliert werden.

22. Verfahren nach einem der vorhergehenden. Ansprüche zur Untersuchung und/oder zum Mustern von Nukleinsäuren oder künstlichen Derivaten von Nukleinsäuren.

23. Verfahren nach einem der vorhergehenden Ansprüche für die Herstellung einer Nukleinsäurebibliothek.

24. Verfahren nach einem der vorhergehenden Ansprüche, wobei es in einem Bioassay eingesetzt wird.

25. Verfahren nach einem der vorhergehenden Ansprüche, **dadurch gekennzeichnet, dass** die Reaktion zur Amplifizierung von Nukleinsäure eine Polymerasekettenreaktion (PCR) ist.

26. Verfahren nach Anspruch 25, umfassend die Schritte
(i) Bereitstellen einer PCR-Reaktionsmischung, bestehend aus Polymerase und/oder dNTPs und/oder Puffersubstanzen und/oder Enhancem und/oder Primern und/oder Templaten und/oder DNA-Sonden und/oder Matrixmaterial,
(ii) Einkapseln der PCR-Reaktionsmischung, so dass dadurch Kapseln gebildet werden, die die Bestandteile der Reaktionsmischung umfassen und
(iii) Ausführen mindestens eines Amplifizierungsschritts, umfassend PCR-Temperaturzyklen, so dass eine Nukleinsäure innerhalb einer oder mehrerer Kapseln amplifiziert wird.

27. Verfahren nach einem der Ansprüche 14-16 oder 25-26, wobei das Templat und/oder einer oder mehrere Primer in die Kapsel transferiert werden, indem eine Veränderung in der Permeabilität der Kapselwand ausgelöst wird.

28. Verfahren nach einem der Ansprüche 14-16 oder 25-26, wobei die Permeabilität der Kapsel reguliert wird, um selektive Diffusion von dNTPs und/oder ddNTPs und/oder kleinen Primern in die Kapseln zu ermöglichen, aber das Herausdiffundieren von Polymerase, PCR-Produkten, Templaten und großen Primern während der Temperaturzyklen zu beschränken.

29. Verfahren nach einem der Ansprüche 25-28, wobei die Kapseln in einem PCR-Puffer dispergiert werden.

30. Verfahren nach Anspruch 29, wobei der PCR-Puffer interkalierende Farbstoffe, wie zum Beispiel Ethidiumbromid und/oder SYBR Green I, enthält.

31. Verfahren nach einem der Ansprüche 25-30, wobei die Temperaturzyklen für die Kapselsuspension in Reagenzgläsern, Multititerplatten oder Glaskapillaren mittels eines Thermocyclers durchgeführt werden.

32. Verfahren nach einem der vorhergehenden Ansprüche, wobei dNTPs und/oder ddNTPs und/oder Primer und/oder Template und/oder DNA-Sonden und/oder Kapselmaterial eine Markierung tragen.

33. Verfahren nach einem der Ansprüche 18-19 oder 32, wobei die Markierung das Kodieren von Primern und/oder Templaten und/oder Mischungen davon ermöglicht.

34. Verfahren nach einem der Ansprüche 25-33, wobei ferner Enzyme, wie zum Beispiel reverse Transkriptasen und/oder Nukleasen und/oder Ligasen in die eingekapselte PCR-Reaktionsmischung gegeben werden.

35. Verfahren zur Sequenzierung des Nukleinsäureinhalts von Kapseln, die durch einen der Ansprüche 1-34 erhalten werden, umfassend die Schritte:
(i) Ausführen der Amplifizierung mittels PCR mit einem Überschuss eines Primers, bis der Primer mit der niedrigeren Konzentration aufgebraucht ist.
(ii) Hinzugeben von mit 4 Farben fluoreszenzmarkierten ddNTPs in einer Menge von 1:100 bis 1:1000 dNTPs und Verlängern des Excess-Primers und
(iii) Untersuchen einzelner Kapseln auf Nukleinsäure, die in der Kapsel enthalten ist, durch Gelelektrophorese.

## Revendications

1. Procédé d'amplification et de détection d'acide nucléique comprenant les étapes
(a) fournir un mélange réactionnel d'amplification d'acide nucléique,
(b) encapsuler ledit mélange réactionnel par 10³ à 10¹² capsules perméables aux molécules de faible masse moléculaire ayant une masse moléculaire inférieure à 2000 Da et non perméables aux molécules de haute masse moléculaire ayant une masse moléculaire supérieure à 2000 Da et former ainsi des capsules comprenant des constituants du mélange réactionnel, et
(c) disperser les capsules dans un tampon comprenant des éléments de construction marqués,
(d) réaliser au moins une étape d'amplification et/ou de polymérisation et amplifier ainsi des acides nucléiques dans une ou plusieurs des capsules contenant une matrice complémentaire et une paire d'amorces, de sorte que des éléments de construction de la réaction d'amplification et/ou de polymérisation traversent et sont introduits dans les capsules pendant la réaction, et accumuler un produit d'acide nucléique dans les capsules, et
(e) détecter des capsules contenant un produit d'acide nucléique au moyen de la signature due au marqueur des éléments de construction marqués.

2. Procédé selon la revendication 1 où les éléments de construction qui sont introduits dans les capsules pendant la réaction sont choisis parmi les dNTP et les ddNTP.

3. Procédé selon l'une quelconque des revendications précédentes où des dNTP marqués ou des ddNTP marqués sont introduits dans les capsules pendant la réaction.

4. Procédé selon l'une quelconque des revendications précédentes où 10⁵ à 10¹¹ amplifications d'acide nucléique sont réalisées en parallèle.

5. Procédé selon l'une quelconque des revendications précédentes où les molécules de haute masse moléculaire qui ne peuvent pas traverser la capsule sont choisies parmi les matrices d'ADN, les produits de PCR et une polymérase.

6. Procédé selon l'une quelconque des revendications précédentes comprenant en outre l'étape
(f) séparer les capsules contenant un produit d'acide nucléique des capsules ne contenant pas de produits d'acide nucléique.

7. Procédé selon l'une quelconque des revendications précédentes comprenant en outre l'étape
(g) analyser ou extraire le produit d'acide nucléique.

8. Procédé selon la revendication 1 où les constituants du mélange réactionnel d'amplification d'acide nucléique sont incorporés dans un matériau de matrice.

9. Procédé selon la revendication 8 où le matériau de matrice est choisi parmi une microparticule poreuse, une substance analogue à une cire, un alginate, un agarose, un polysaccharide, un polypeptide, un acide gras, un sel d'un acide gras, la polyvinylpyrrolidone (PVP), ou la matrice est la glace, ou un mélange de ceux-ci.

10. Procédé selon l'une quelconque des revendications précédentes où le mélange réactionnel d'amplification d'acide nucléique incorporé dans un matériau de matrice est encapsulé au moyen d'un procédé couche par couche, d'un procédé de polymérisation et/ou d'un procédé de cavaciation.

11. Procédé selon l'une quelconque des revendications 8 à 10 où le matériau de matrice est dissous et/ou retiré de la capsule avec des solvants, par des moyens chimiques ou par des moyens physiques comme la chaleur.

12. Procédé selon l'une quelconque des revendications 8 à 10 où le matériau de matrice est fondu pendant le parcours de cycles de température.

13. Procédé selon l'une quelconque des revendications précédentes où des capsules ayant un diamètre de 50 nm à 1000 µm, avec un diamètre préféré de 40 à 60 µm, sont formées.

14. Procédé selon l'une quelconque des revendications précédentes où des constituants et/ou réactifs du mélange réactionnel d'amplification d'acide nucléique sont transférés dans la capsule par un changement déclenché de la perméabilité de la paroi de la capsule.

15. Procédé selon la revendication 14 où le changement de perméabilité de la paroi de la capsule est déclenché par un changement de pH ou un changement de force ionique ou un changement de réticulation de la capsule.

16. Procédé selon la revendication 14 ou 15 où la perméabilité de la capsule est contrôlée pour permettre la diffusion sélective de produits de départ de la réaction d'amplification d'acide nucléique dans les capsules mais pour limiter la diffusion à l'extérieur de produits réactionnels d'amplification d'acide nucléique et/ou de matrices et/ou d'amorces.

17. Procédé selon l'une quelconque des revendications précédentes où des composés du mélange réactionnel d'amplification d'acide nucléique non encapsulés sont séparés des capsules ou détruits par des enzymes avant l'étape d'amplification.

18. Procédé selon l'une quelconque des revendications précédentes où un ou plusieurs composants du mélange réactionnel d'amplification d'acide nucléique et/ou du matériau de la capsule portent un marqueur.

19. Procédé selon la revendication 18 où le marqueur est choisi parmi un fluorophore, un point quantique, un radioisotope, un colorant, une nanoparticule ou un isotope actif en RMN.

20. Procédé selon la revendication 18 ou 19 où le marqueur permet le codage de capsules individuelles.

21. Procédé selon l'une quelconque des revendications précédentes où les capsules contenant des produits d'acide nucléique sont détectées par la signature physique due à un marqueur ou par une mesure d'ADN par adsorption UV, ou par des colorants colorant l'ADN, et sont séparées et isolées de la suspension de capsules.

22. Procédé selon l'une quelconque des revendications précédentes pour l'analyse et/ou le criblage d'acides nucléiques ou de dérivés artificiels d'acides nucléiques.

23. Procédé selon l'une quelconque des revendications précédentes pour la production d'une banque d'acide nucléique.

24. Procédé selon l'une quelconque des revendications précédentes employé dans une bioanalyse.

25. Procédé selon l'une quelconque des revendications précédentes **caractérisé en ce que** la réaction d'amplification d'acide nucléique est une réaction en chaîne par polymérase (PCR).

26. Procédé selon la revendication 25 comprenant les étapes
(i) fournir un mélange réactionnel de PCR consistant en polymérase et/ou dNTP et/ou substances tampons et/ou activateurs et/ou amorces et/ou matrices et/ou sondes d'ADN et/ou matériau de matrice,
(ii) encapsuler ledit mélange réactionnel de PCR et former ainsi des capsules comprenant des constituants du mélange réactionnel et
(iii) réaliser au moins une étape d'amplification comprenant le parcours de cycles de température de PCR et amplifier ainsi un acide nucléique dans une ou plusieurs des capsules.

27. Procédé selon l'une quelconque des revendications 14-16 ou 25-26 où la matrice et/ou une ou plusieurs amorces sont transférées dans la capsule par un changement déclenché de la perméabilité de la paroi de la capsule.

28. Procédé selon l'une quelconque des revendications 14-16 ou 25-26 où la perméabilité de la capsule est contrôlée pour permettre la diffusion sélective de dNTP et/ou de ddNTP et/ou de petites amorces dans les capsules mais pour limiter la diffusion vers l'extérieur de polymérase, de produits de PCR, de matrices et de grandes amorces pendant le parcours des cycles de température.

29. Procédé selon l'une quelconque des revendications 25-28 où les capsules sont dispersées dans un tampon de PCR.

30. Procédé selon la revendication 29 où le tampon de PCR contient des colorants intercalants comme le bromure d'éthidium et/ou le SYBR Green I.

31. Procédé selon l'une quelconque des revendications 25-30 où le parcours de cycles de température de la suspension de capsules est réalisé dans des tubes de réaction, des plaques de multititrage ou des capillaires en verre, au moyen d'un thermocycleur.

32. Procédé selon l'une quelconque des revendications précédentes où les dNTP et ou les ddNTP et/ou les amorces et/ou les matrices et/ou les sondes d'ADN et/ou le matériau des capsules portent un marqueur.

33. Procédé selon l'une quelconque des revendications 18-19 ou 32 où le marqueur permet le codage d'amorces et/ou de matrices et/ou de mélanges de celles-ci.

34. Procédé selon l'une quelconque des revendications 25-33 où d'autres enzymes comme des transcriptases inverses et/ou des nucléases et/ou des ligases sont ajoutées dans le mélange réactionnel de PCR encapsulé.

35. Procédé de séquençage du contenu en acide nucléique de capsules obtenues par l'une quelconque des revendications 1-34 comprenant les étapes :
(i) réaliser l'amplification par PCR avec un excès d'une amorce jusqu'à ce que l'amorce ayant la plus basse concentration soit consommée,
(ii) ajouter des ddNTP marqués par fluorescence à 4 couleurs à une quantité de 1: 100 à 1 : 1000 de dNTP et soumettre à une élongation l'amorce en excès et
(iii) analyser le contenu de capsules en acide nucléique de capsules individuelles par électrophorèse en gel.
